# EUROPÄISCHE PATENTSCHRIFT

(11) **EP 2 207 490 B1**
(45) Veröffentlichungstag und Bekanntmachung des Hinweises auf die Patenterteilung: **12.03.2014**
(21) Anmeldenummer: 08848108.0
(22) Anmeldetag: 04.11.2008
(51) Int. Cl.: A61B 17/3203

(54) **WASSERSTRAHLCHIRURGIEGERÄT UND VERFAHREN ZUM BETRIEB EINES SOLCHEN**
SURGICAL DEVICE USING WATER JET AND METHOD FOR OPERATING SAID DEVICE
APPAREIL CHIRURGICAL À JET D'EAU ET PROCÉDÉ POUR SON UTILISATION

(30) Priorität: 06.11.2007 DE 102007052805
(43) Veröffentlichungstag der Anmeldung: 21.07.2010
(60) Teilanmeldung: 13166590.3
(73) Patentinhaber: ERBE Elektromedizin GmbH, 72072 Tübingen (DE)
(72) Erfinder: WAHL, Hans-Jürgen, 72818 Trochtelfingen (DE); PFÄFFLE, Alexander, 72810 Gomaringen (DE); KÜHNER, Ralf, 70567 Stuttgart (DE)
(74) Vertreter: Bohnenberger, Johannes
(86) Internationale Anmeldenummer: PCT/EP2008/009290
(87) Internationale Veröffentlichungsnummer: WO 2009/059742

(56) Entgegenhaltungen:
- EP-A- 0 879 578
- US-A- 4 655 742
- US-A- 5 536 242
- US-A1- 2005 059 924
- US-A1- 2006 156 875
- US-A1- 2006 247 743

## Beschreibung

Die Erfindung betrifft ein Wasserstrahlchirurgiegerät nach dem Oberbegriff des Patentanspruches 1 sowie ein Verfahren zum Betreiben eines solchen.

Wasserstrahlchirurgiegeräte, mittels derer Gewebe durch einen Hochdruck-Wasserstrahl getrennt werden kann, sind allgemein bekannt. Wenn man ein solches Gerät in der offenen Chirurgie benutzt, so ist das Abführen der "Schneidflüssigkeit" unproblematisch. Bei Verwendung derartiger Geräte bei endoskopischen Operationen, also innerhalb von Körperhöhlen, können durch die Zufuhr der Schneidflüssigkeit Probleme auftreten.

EP-A-879 578 offenbart ein Gerät gemäß dem Oberbegriff des Anspruchs 1.

Der Erfindung liegt die Aufgabe zugrunde, ein Wasserstrahlchirurgiegerät der eingangs genannten Art dahingehend aufzuzeigen, dass die insbesondere bei endoskopischen Operationen auftretenden Probleme vermindert werden.

Diese Aufgabe wird durch ein Wasserstrahlchirurgiegerät nach Anspruch 1 bzw. ein Verfahren nach Anspruch 8 gelöst. Insbesondere wird diese Aufgabe durch ein Wasserstrahlchirurgiegerät gelöst, umfassend eine Flüssigkeitsfördereinrichtung, die zur Abgabe einer Flüssigkeit in einer Anschlussleitung eines Chirurgieinstruments mit einer Auslassdüse durch Ansteuersignale aus einer Steuereinrichtung ansteuerbar ist, wobei mindestens eine Messeinrichtung vorgesehen und derart ausgebildet ist, dass nach einem Anschließen des Chirurgieinstruments an die Flüssigkeitsfördereinrichtung die Messeinrichtung Messsignale zur Darstellung einer Menge abgegebener Flüssigkeit in einer Anzeige- oder Registriereinheit erzeugt. Dadurch ist es möglich, bei einer Operation die in eine Körperhöhle eingeführte Schneidflüssigkeit ihrer Menge nach zu bestimmen und mit der Flüssigkeitsmenge zu vergleichen, die vom Operationsfeld abgesaugt wird. Darüber hinaus ist es möglich, eine exakte Planung der Operation vorzunehmen und zwar hinsichtlich der verbrauchten Menge an Schneidflüssigkeit, die in einem Vorratsbehälter vorgehalten wird.

Die Messeinrichtung umfasst vorzugsweise einen Füllsensor und erzeugt ein Füllsignal dann, wenn die Anschlussleitung des Chirurgieinstruments im Wesentlichen bis zur Auslassdüse gefüllt ist und gibt eine zum Füllen der Anschlussleitung benötigte Füllmenge wieder. Auf diese Weise kann das Wasserstrahlchirurgiegerät in dem Sinne funktionsbereit gemacht werden, dass der Operateur dann, wenn er ein Startsignal zum Beginn eines Schneidvorgangs gibt, tatsächlich Schneidflüssigkeit an der Auslassdüse ansteht.

Die Anzeige- oder Registriereinheit ist derart ausgebildet, dass die Menge abgegebener Flüssigkeit abzüglich der Füllmenge angezeigt wird. Diese Füllmenge kann besonders bei langen Anschlussleitungen oder Chirurgieinstrumenten mit einer hohen Flüssigkeitskapazität relativ beträchtlich sein, sodass die Messung der in den Körper abgegebenen Flüssigkeitsmenge verfälscht wird.

Vorzugsweise ist eine Eingabevorrichtung vorgesehen, über welche die Steuereinrichtung ein dem angeschlossenen Chirurgieinstrument entsprechendes Mengensignal zur Abgabe einer von der Flüssigkeitsfördereinrichtung abzugebenden Flüssigkeitsmenge zuführbar ist. Bei dieser Ausführungsform der Erfindung muss also nicht unbedingt gemessen werden, wann die Anschlussleitung bzw. das Chirurgieinstrument befüllt ist, es wird hier vielmehr eine Menge von Schneidflüssigkeit vorgegeben, die von der Flüssigkeitsfördereinrichtung abgegeben wird, sodass man sich dann sicher sein kann, dass das Gerät befüllt ist.

Diese Eingabe der Flüssigkeitsmenge kann durch eine manuell bedienbare Eingabevorrichtung vorgegeben werden. Bei einer anderen bevorzugten Ausführungsform der Erfindung wird die zum Befüllen des Chirurgieinstrumentes bzw. seiner Anschlussleitung notwendige Flüssigkeitsmenge direkt durch eine Codiereinrichtung im Chirurgieinstrument der Eingabevorrichtung übermittelt, sodass ein manuelles Programmieren entfällt.

Wenn ein Füllsensor vorhanden ist, so kann dieser als Drucksensor ausgebildet sein, der bei einer vorbestimmten Druckänderung oder einem vorbestimmten zeitlichen Verlauf des Druckes das Füllsignal erzeugt. Der Druck der Schneidflüssigkeit ist nämlich niedrig, solange aus der Auslassdüse Luft herausgedrückt wird.

Bei einer anderen Ausführungsform der Erfindung umfasst die Sensoreinrichtung einen Feuchtigkeits- oder Leitfähigkeitssensor, der vorzugsweise sehr nahe an der Auslassdüse angebracht ist und dann ein Signal erzeugt, wenn die Flüssigkeit tatsächlich an diesem Ort angekommen ist.

Verfahrensmäßig umfasst das Verfahren zum Betreiben eines Wasserstrahlchirurgiegerätes die folgenden Schritte:
- Anschließen eines Chirurgieinstruments an das Wasserstrahlchirurgiegerät;
- Erzeugen von Ansteuersignalen zum Ansteuern einer Flüssigkeitsfördereinrichtung derart, dass diese Flüssigkeit solange dem Chirurgieinstrument zuführt, bis dieses im Wesentlichen bis zu seiner Auslassdüse gefüllt ist;
- Beenden der Ansteuersignale und Erzeugen eines Betriebsfreigabesignals derart, dass eine Ansteuerung der Flüssigkeitsfördereinrichtung zum Operationsbetrieb des Wasserstrahlchirurgiegeräts zugelassen wird;
- Anzeigen einer Menge abgegebener Flüssigkeit abzüglich einer zum Füllen des Chirurgieinstruments benötigten Füllmenge auf einer Anzeige- oder Registriereinheit.

Es wird also in diesem Fall eine Betätigung des Wasserstrahlchirurgiegeräts unter Operationsbedingungen verhindert, bis das Chirurgieinstrument wirklich "startklar" ist.

Nachfolgend wird eine Ausführungsform der Erfindung anhand von Abbildungen näher erläutert. Hierbei zeigen
- - Fig. 1: eine schematische Darstellung zur Erläuterung der verschiedenen Baugruppen und
- - Fig. 2: ein Diagramm zur Erläuterung des Druckverlaufes beim Befüllen des Chirurgieinstruments.

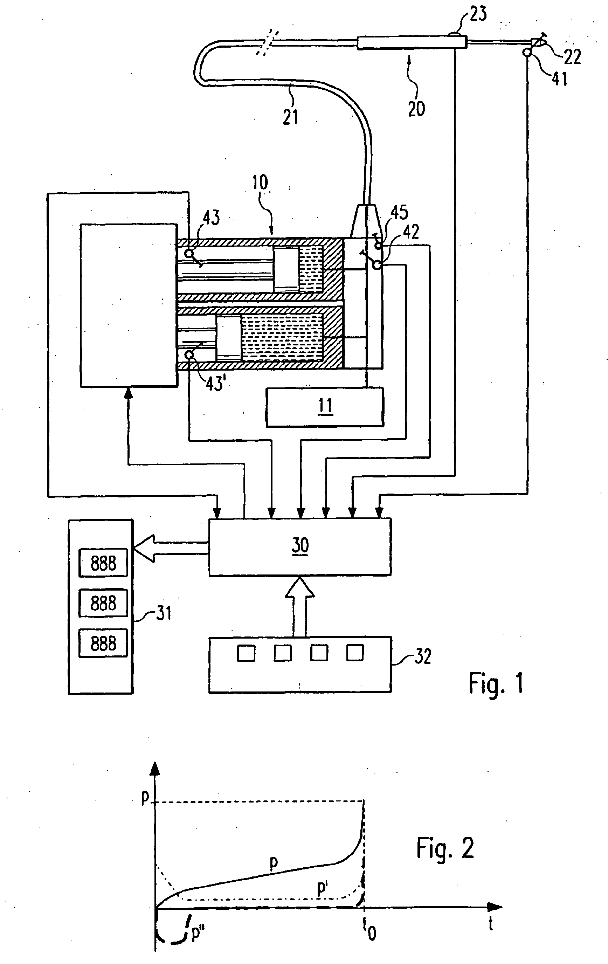

In Fig. 1 ist als Flüssigkeitsfördereinrichtung 10 eine Pumpe mit zwei Kolben dargestellt, die aus einem Vorratsbehälter 11 Schneidflüssigkeit über eine Anschlussleitung 21 eines Chirurgieinstrumentes 20 bis zu einer Auslassdüse 22 fördert, aus welcher bei Betätigung einer Bedienungstaste 23 Schneidflüssigkeit aus der Auslassdüse 22 zum Trennen von Gewebe oder auch zum Injizieren in ein Gewebe ausgestoßen wird. Selbstverständlich sind auch andere Druckerzeugungseinrichtungen verwendbar.

Die Kolben der bei diesem Ausführungsbeispiel gezeigten Flüssigkeitsfördereinrichtung werden hinsichtlich ihrer Position über Positionssensoren 43, 43' überwacht, deren Ausgangssignale einer Steuereinrichtung 30 zugeführt werden.

Weiterhin ist an der Flüssigkeitsfördereinrichtung 10 ein Ankoppelfühler 45 vorgesehen, der dann anspricht, wenn ein Chirurgieinstrument 20 mit der Flüssigkeitsfördereinrichtung 10 verbunden wird.

Der von der Flüssigkeitsfördereinrichtung 10 erzeugte Druck wird mittels eines Drucksensors 42 abgetastet, dessen Ausgangssignale der Steuereinrichtung 30 zugeführt werden. Alternativ oder zusätzlich kann ein Feuchtesensor 41 in der Auslassdüse 22 oder zumindest in deren Nähe vorgesehen sein, der dann anspricht, wenn Schneidflüssigkeit bis zu diesem Punkt gelangt ist.

Die Steuereinrichtung 30 ist mit einer Anzeigeeinheit 31 verbunden, auf welcher die verschiedenen Parameter, insbesondere aber die von der Flüssigkeitsfördereinrichtung 10 geförderte und aus der Auslassdüse 22 ausgestoßene Flüssigkeitsmenge angezeigt wird.

Weiterhin ist eine Eingabevorrichtung 32 vorgesehen, über welche der Steuereinrichtung 30 Betriebsdaten eingegeben werden können, wobei diese Betriebsdaten zum Beispiel die Flüssigkeitsmenge wiedergeben, welche in das Chirurgieinstrument 20 zu fördern ist, wenn dieses bis zu seiner Auslassdüse 22 hin gefüllt werden soll.

Wenn also ein Chirurgieinstrument 20 an die Flüssigkeitsfördereinrichtung 10 angesteckt wird, so erzeugt der Ankoppelfühler 45 ein entsprechendes Signal, welches der Steuereinrichtung 30 mitgeteilt wird. Auf ein entsprechendes "Füllkommando" hin, welches der Operateur über die Eingabevorrichtung 32 eingibt, arbeitet nun die Flüssigkeitsfördereinrichtung 10 solange, bis das Chirurgieinstrument 20 bis zu seiner Auslassdüse 22 hin mit Schneidflüssigkeit gefüllt ist. Danach kann der Operateur das Gerät durch Betätigen der Bedienungstaste 23 aktivieren, also einen Strahl von Schneidflüssigkeit aus der Auslassdüse 22 derart austreten lassen, dass ein anvisiertes Zielgewebe durchtrennt wird. Die während der Operation abgegebene und tatsächlich aus der Auslassdüse 22 ausgetretene Flüssigkeitsmenge, also die Gesamtfördermenge abzüglich der im Chirurgieinstrument 20 enthaltenen Flüssigkeitsmenge wird auf der Anzeigeeinrichtung 31 angezeigt.

In Fig. 2 ist ein Druckverlauf idealisiert dargestellt. Der Druck p steigt nach diesem Diagramm an, während die Schneidflüssigkeit in die Anschlussleitung in Richtung auf die Auslassdüse 22 strömt, da das Luftpolster, welches die Flüssigkeit vor sich her treibt, immer kleiner wird. In dem Moment (t-Index 0), in welchem die Schneidflüssigkeit die Auslassdüse 22 erreicht, steigt der Druck abrupt an, da die Schneidflüssigkeit aufgrund ihrer gegenüber Luft sehr hohen Viskosität einen höheren Strömungswiderstand an der (sehr kleinen) Auslassdüse 22 aufweist. Dieser abrupte Druckanstieg kann dazu benützt werden, ein Signal abzugeben, welches anzeigt, dass nun das Chirurgieinstrument 20 korrekt gefüllt und somit betriebsbereit ist. Noch leichter ist die zweite Ableitung des Druckes nach der Zeit zum Generieren eines solchen Signals geeignet, da diese nur dann einen positiven Wert aufweist, wenn der genannte steile Druckanstieg tatsächlich vorliegt.

### Bezugszeichenliste

- 10: Flüssigkeitsfördereinrichtung
- 11: Vorratsbehälter
- 20: Chirurgieinstrument
- 21: Anschlussleitung
- 22: Auslassdüse
- 23: Bedienungstaste
- 30: Steuereinrichtung
- 31: Anzeigeeinheit
- 32: Eingabevorrichtung
- 41: Feuchtesensor
- 42: Drucksensor
- 43, 43': Positionssensor
- 45: Ankoppelfühler

## Patentansprüche

1. Wasserstrahlchirurgiegerät, umfassend
eine Flüssigkeitsfördereinrichtung (10), die zur Abgabe einer Flüssigkeit in eine Anschlussleitung (21) eines Chirurgieinstruments (20) mit einer Auslassdüse (22) durch Ansteuersignale aus einer Steuereinrichtung (30) ansteuerbar ist,
wobei mindestens eine Messeinrichtung (41 - 43) vorgesehen und derart ausgebildet ist, dass nach einem Anschließen des Chirurgieinstruments (20) an die Flüssigkeitsfördereinrichtung (10) die Messeinrichtung (41 - 43) Messsignale zur Darstellung einer Menge abgegebener Flüssigkeit in einer Anzeige- oder Registriereinheit (31) erzeugt,
**dadurch gekennzeichnet, dass**
die Anzeige- oder Registriereinheit (31) derart ausgebildet ist, dass die Menge abgegebener Flüssigkeit abzüglich einer Füllmenge angezeigt wird, die zum Füllen der Anschlussleitung (21) benötigt wird.

2. Wasserstrahlchirurgiegerät nach Anspruch 1,
**dadurch gekennzeichnet, dass**
die Messeinrichtung (41, 42) einen Füllsensor umfasst und ein Füllsignal dann erzeugt, wenn die Anschlussleitung (21) im Wesentlichen bis zur Auslassdüse (22) gefüllt ist und die zum Füllen der Anschlussleitung (21) benötigte Füllmenge wiedergibt.

3. Wasserstrahlchirurgiegerät nach einem der vorhergehenden Ansprüche,
**dadurch gekennzeichnet, dass**
eine Eingabevorrichtung (32) vorgesehen ist, über welche der Steuereinrichtung (30) ein dem angeschlossenen Chirurgieinstrument (20) entsprechendes Mengensignal zur Eingabe einer von der Flüssigkeitsfördereinrichtung (10) abzugebenden Flüssigkeitsmenge zuführbar ist.

4. Wasserstrahlchirurgiegerät nach einem der vorhergehenden Ansprüche, insbesondere nach Anspruch 3,
**dadurch gekennzeichnet, dass**
die Eingabevorrichtung (32) manuell bedienbar ist.

5. Wasserstrahlchirurgiegerät nach einem der vorhergehenden Ansprüche, insbesondere nach Anspruch 3,
**dadurch gekennzeichnet, dass**
die Eingabevorrichtung (32) durch das Chirurgieinstrument (10) programmierbar ist.

6. Wasserstrahlchirurgiegerät nach einem der vorhergehenden Ansprüche, insbesondere nach Anspruch 2,
**dadurch gekennzeichnet, dass**
der Füllsensor einen Drucksensor (42) umfasst, der bei einer vorbestimmten Druckänderung oder einem vorbestimmten zeitlichen Verlauf des Druckes das Füllsignal erzeugt.

7. Wasserstrahlchirurgiegerät nach einem der vorhergehenden Ansprüche, insbesondere nach Anspruch 2,
**dadurch gekennzeichnet, dass**
die Sensoreinrichtung einen Feuchtigkeits- oder Leitfähigkeitssensor (41) umfasst.

8. Verfahren zum Betreiben eines Wasserstrahlchirurgiegerätes, umfassend die Schritte
- Anschließen eines Chirurgieinstruments an das Wasserstrahlchirurgiegerät;
- Erzeugen von Ansteuersignalen zum Ansteuern einer Flüssigkeitsfördereinrichtung derart, dass diese Flüssigkeit so lange dem Chirurgieinstrument zuführt, bis dieses im Wesentlichen bis zu seiner Auslassdüse gefüllt ist;
- Beenden der Ansteuersignale und Erzeugen eines Betriebsfreigabesignals derart, dass eine Ansteuerung der Flüssigkeitsfördereinrichtung zum Operationsbetrieb des Wasserstrahichirurgiegerätes zugelassen wird;
- Anzeigen einer Menge abgegebener Flüssigkeit abzüglich einer zum Füllen des Chirurgieinstruments benötigten Füllmenge auf einer Anzeige- oder Registriereinheit.

## Claims

1. Surgical device using a water jet, comprising
a liquid supply apparatus (10), which can be actuated by actuation signals from a control apparatus (30) for dispensing a liquid into a connection line (21) of a surgical instrument (20) with an outlet nozzle (22),
wherein at least one measuring apparatus (41-43) is provided and configured in such a way that, after connecting the surgical instrument (20) to the liquid supply apparatus (10), the measuring apparatus (41-43) generates measurement signals for indicating an amount of liquid dispensed, in a display or registration unit (31), **characterized in that**
the display or registration unit (31) is configured in such a way that what is displayed is the amount of liquid dispensed, minus a filling amount required for filling the connection line (21).

2. Surgical device using a water jet, according to Claim 1,
**characterized in that**
the measuring apparatus (41, 42) comprises a filling sensor and generates a fill signal if the connection line (21) is substantially filled to the outlet nozzle (22) and reproduces the filling amount required for filling the connection line (21).

3. Surgical device using a water jet, according to one of the preceding claims,
**characterized in that**
an input means (32) is provided, by means of which an amount signal corresponding to the connected surgical instrument (20) can be supplied to the control apparatus (30) for entering an amount of liquid to be dispensed by the liquid supply apparatus (10).

4. Surgical device using a water jet, according to one of the preceding claims, in particular according to Claim 3,
**characterized in that**
the input means (32) can be operated manually.

5. Surgical device using a water jet, according to one of the preceding claims, in particular according to Claim 3,
**characterized in that**
the input means (32) can be programmed by the surgical instrument (20).

6. Surgical device using a water jet, according to one of the preceding claims, in particular according to Claim 2,
**characterized in that**
the filling sensor comprises a pressure sensor (42) which generates the fill signal in the case of a predetermined pressure change or a predetermined time profile of the pressure.

7. Surgical device using a water jet, according to one of the preceding claims, in particular according to Claim 2,
**characterized in that**
the sensor apparatus comprises a humidity or conductivity sensor (41).

8. Method for operating a surgical device using a water jet, comprising the following steps:
- connecting a surgical instrument to the surgical device using a water jet;
- generating actuation signals for actuating a liquid supply apparatus in such a way that the latter supplies liquid to the surgical instrument until the latter is filled substantially up to the outlet nozzle thereof;
- terminating the actuation signals and generating an operation release signal in such a way that actuation of the liquid supply apparatus for surgical operation of the surgical device using a water jet is enabled;
- displaying an amount of liquid dispensed, minus a filling amount required for filling the surgical instrument, on a display or registration unit.

## Revendications

1. Appareil chirurgical à jet d'eau, comprenant un dispositif d'apport de liquide (10) qui peut être commandé pour délivrer un liquide dans une conduite de raccordement (21) d'un instrument chirurgical (20) comportant une buse de sortie (22) au moyen de signaux de commande provenant d'un dispositif de commande (30), dans lequel il est prévu au moins un dispositif de mesure (41 - 43) qui est conçu de manière à ce que, après un raccordement de l'instrument chirurgical (20) au dispositif d'apport de liquide (10), le dispositif de mesure (41 - 43) génère des signaux de mesure destinés à représenter une quantité de liquide délivré dans une unité d'affichage ou d'enregistrement (31), **caractérisé en ce que** l'unité d'affichage ou d'enregistrement (31) est conçue de manière à ce que la quantité de liquide délivré soit affichée après soustraction d'une quantité de remplissage qui est nécessaire pour le remplissage de la conduite de raccordement (21).

2. Appareil chirurgical à jet d'eau selon la revendication 1, **caractérisé en ce que** le dispositif de mesure (41, 42) comprend un capteur de remplissage et ne génère un signal de remplissage que lorsque la conduite de raccordement (21) est remplie sensiblement jusqu'à la buse de sortie (22) et **en ce qu'**il indique la quantité de remplissage nécessaire pour le remplissage de la conduite de raccordement (21).

3. Appareil chirurgical à jet d'eau selon l'une quelconque des revendications précédentes, **caractérisé en ce qu'**il est prévu un dispositif d'entrée (32) au moyen duquel un signal de quantité correspondant à un instrument chirurgical raccordé (20) peut être délivré à l'unité de commande (30) pour la fourniture en entrée d'une quantité de liquide devant être délivrée par le dispositif d'apport de liquide (10).

4. Appareil chirurgical à jet d'eau selon l'une quelconque des revendications précédentes, en particulier selon la revendication 3, **caractérisé en ce que** le dispositif d'entrée (32) peut être actionné manuellement.

5. Appareil chirurgical à jet d'eau selon l'une quelconque des revendications précédentes, en particulier selon la revendication 3, **caractérisé en ce que** le dispositif d'entrée (32) peut être programmé par l'intermédiaire de l'instrument chirurgical (20).

6. Appareil chirurgical à jet d'eau selon l'une quelconque des revendications précédentes, en particulier selon la revendication 2, **caractérisé en ce que** le capteur de remplissage comprend un capteur de pression (42) qui génère le signal de remplissage lors d'une variation de pression prédéterminée ou d'une évolution temporelle prédéterminée de la pression.

7. Appareil chirurgical à jet d'eau selon l'une quelconque des revendications précédentes, en particulier selon la revendication 2, **caractérisé en ce que** le dispositif de détection comprend un capteur d'humidité ou de conductivité (41).

8. Procédé de mise en fonctionnement d'un appareil chirurgical à jet d'eau, comprenant les étapes suivantes :
- raccordement d'un instrument chirurgical à l'appareil chirurgical à jet d'eau ;
- génération de signaux de commande destinés à commander un dispositif d'apport de liquide de telle manière que ledit liquide soit introduit dans l'instrument chirurgical jusqu'à ce que celui-ci soit sensiblement rempli jusqu'à sa buse de sortie ;
- interruption du signal de commande et de la génération d'un signal de déclenchement du fonctionnement de manière à autoriser une commande du dispositif d'apport de liquide pour la mise en fonctionnement de l'appareil chirurgical à jet d'eau ;
- affichage sur une unité d'affichage ou d'enregistrement d'une quantité de liquide délivrée après soustraction d'une quantité de remplissage nécessaire pour le remplissage de l'instrument chirurgical.
